# EUROPÄISCHE PATENTSCHRIFT

(11) **EP 0 421 161 B1**
(45) Veröffentlichungstag und Bekanntmachung des Hinweises auf die Patenterteilung: **05.07.1995**
(21) Anmeldenummer: 90117551.3
(22) Anmeldetag: 12.09.1990
(51) Int. Cl.: A61B 17/12

(54) **Klemme zum Abklemmen von Blutgefässen und Aneurysmen**
Clip for blood vessels and aneurysms
Pince pour vaisseaux sanguins et anévrismes

(30) Priorität: 06.10.1989 DE 8911948 U
(43) Veröffentlichungstag der Anmeldung: 10.04.1991
(73) Patentinhaber: von Zeppelin, Dieter, D-82049 Pullach (DE)
(72) Erfinder: von Zeppelin, Dieter, D-82049 Pullach (DE)
(74) Vertreter: Müller-Boré & Partner Patentanwälte

(56) Entgegenhaltungen:
- EP-A- 0 105 414
- DE-A- 3 723 167
- DE-B- 2 658 478

## Beschreibung

Die Erfindung betrifft eine Klemme zum Abklemmen von Blutgefäßen oder Aneurismen, bestehend aus zwei ein Maulteil und eine Bedienungsbranche aufweisenden Armen, welche mit einer die Maulteile gegeneinander drängenden, an den Bedienungsbranchen befestigten Schließfeder verbunden sind, wobei die Arme einander schwenklagerfrei überkreuzen und die Schließfeder eine Schraubenfeder mit wenigstens einer Windung ist, deren verlängerte Federenden je mit einer der beiden Innenflächen der Bedienungsbranchen fest verbunden sind.

In der Chirurgie, vorzugsweise der Mikrochirurgie, müssen Blutgefäße, Aneurysmen und dergleichen in oft räumlich sehr beengter Umgebung abgeklemmt werden. Besonders groß ist dieses Problem bei tief liegenden kraniellen Aneurysmen, welche durch umgebende Strukturen die räumlichen Verhältnisse und Sichtverhältnisse beeinträchtigen.

Es ist eine Klemme der vorgenannten Art bekannt (Prospektblatt der Firma Ab. KRAUTH "Heifetz Intrakraniale Aneurisma Clips und Setzstangen"), bei welcher die Arme einander nicht überkreuzen. Zum Anlegen der Klemme werden deshalb die Bedienungsbranchen von einer Setzstange von außen umgriffen, wodurch Teile der Klemme verdeckt werden. Das Umgreifen der Klemme von außen bedingt vergleichsweise große Abmessungen der Setzstange im Greifbereich, wodurch die Sicht auf die Klemme und auf das abzuklemmende Gefäß bzw. die abzuklemmenden Aneurysmen stark behindert wird. Im übrigen sind die Bedienungsbranchen der Klemme mit einem geradlinig verlaufenden Vorsprung bzw. einer entsprechenden Vertiefung versehen, weshalb die Klemme in Bezug auf die Setzstange nicht verdrehbar ist, weshalb eine große Anzahl verschiedener Klemmen erforderlich ist.

Es sind auch Aneurisma- und Gefäßclips der eingangs genannten Art bekannt (DE-B-26 58 478). Diese Clips weisen auch sich schwenklagerfrei überkreuzende Arme auf. Sie sind aber einschließlich der Schließfeder aus einem Stück federnden Stahles geformt, weshalb auch hier zum öffnen des Maulteiles die Bedienungsbranche eines Clips von einem Anlegeinstrument umgriffen und zusammengedrückt werden muß, weshalb hier die gleichen Nachteile durch Sichtbehinderung auftreten, wie sie oben beschrieben sind.
Der Prospekt der Firma AESCULAP, S-033 981/4, Auflage 1 "MICRO Aneurisma- und Gefäßclips", zeigt auch Clips mit verbreiterten Bedienungsbranchen, in denen Bohrungen zur Aufnahme eines Zapfens am Anlegeinstrument vorgesehen sind, weshalb der Clip in dem Anlegeinstrument seitlich verschwenkbar ist. Die Probleme der Sichtbehinderung sind aber auch hier vorhanden.

Es ist auch eine Klemme der eingangs genannten Art bekannt DE-A-37 23 167, die den gegebenen engen räumlichen Verhältnissen Rechnung trägt und die mit vergleichsweise geringer Sichtbehinderung durch das Anlegeinstrument anlegbar ist, bei welcher aber die aus vollem Material herausgearbeiteten Arme um einen zwischen Maulteil und Bedienungsbranche liegenden Stift o. dgl. schwenkbar gelagert sind. Die Fabrikation dieser bekannten Klemme ist mit Rücksicht auf die aufwendige Herstellung der Arme und die erforderliche paßgenaue Lagerstelle verhältnismäßig aufwendig.

Der Erfindung liegt die Aufgabe zugrunde, eine Klemme der eingangs genannten Art zu schaffen, die bei geringer Sichtbehinderung eine einfache Herstellung ermöglicht.

Dies wird erfindungsgemäß dadurch erreicht, daß die Bedienungsbranchen mit Rastelementen in Form von Vorsprüngen oder Vertiefungen für ein Anlegeinstrument an den einander zugewandten Seiten der Bedienungsbranchen versehen sind und daß die Schließfeder zwischen den Bedienungsbranchen liegt und mit ihren verlängerten Federenden je mit einer der beiden Innenflächen der Bedienungsbranchen verbunden sind.

Dabei ist das Anlegeinstrument für die Klemme von der Innenseite an die Bedienungsbranchen anlegbar, wodurch eine geringe Sichtbehinderung erreichbar ist. Die Klemme läßt sich in einfacher Weise aus biegbarem Material mit verhältnismäßig geringem Kostenaufwand herstellen.

Die Schließfeder der Klemme kann beispielsweise so ausgebildet sein, daß sich die Enden der Schließfeder im Anschluß an die Federwindung und vor der Befestigung an den Bedienungsbranchen überkreuzen.

Die erfindungsgemäße Klemme ist beim Anlegen weitgehend in ganzer Größe sichtbar. Auch öffnen sich die Maulteile weitgehend auf ihrer ganzen Länge.

Die Erfindung ist im folgenden anhand der Zeichnung an Ausführungsbeispielen näher erläutert. In der Zeichnung zeigen:
- Fig.1: Eine Draufsicht auf eine erste Ausführung nach der Erfindung in etwa 5-facher Vergrößerung,
- Fig.2: eine Seitenansicht der Klemme nach Fig.1 und
- Fig.3: eine Draufsicht auf eine weitere Ausführung nach der Erfindung.

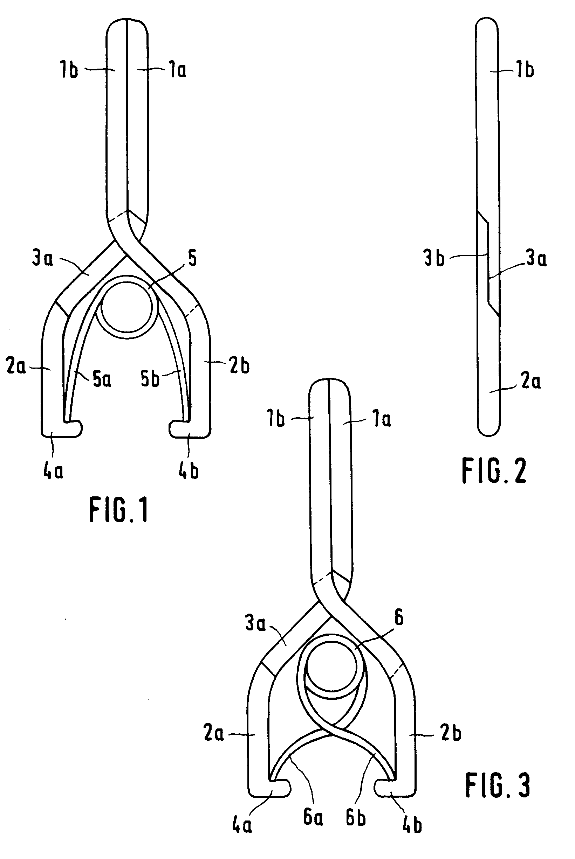

Wie in den Figuren 1 und 2 gezeigt, besteht die Klemme aus zwei einander überkreuzenden Armen, die je ein Maulteil 1a,1b und eine Bedienungsbranche 2a,2b aufweisen. Die Arme sind zweckmäßig aus einem Drahtmaterial hergestellt, das in die entsprechende Form gebogen werden kann. Im Überkreuzungsbereich 3a,3b der Arme sind diese ausgenommen, so daß die Maulteile 1a,1b aufeinander liegen.

Zwischen den Bedienungsbranchen 2a,2b ist die Schließfeder in Form einer Schraubenfeder (5) mit wenigstens einer Windung angeordnet, deren Federenden 5a,5b je an einer Innenseite der Branchen 2a,2b befestigt sind.

Die freien Enden 4a,4b der Bedienungsbranchen 2a,2b weisen nach innen gerichtete Rastelemente für ein Anlegeinstrument auf, mit dessen Hilfe die Maulteile 1a,1b der Klemme durch Auseinanderbewegen der Bedienungsbranchen 2a,2b geöffnet werden können. Bei einer solchen Öffnungsbewegung schwenken die beiden Arme um die Mittelachse der Schraubenfeder 5, ohne daß ein besonderes, paßgenaues Schwenklager erforderlich ist.

Die Rastelemente 4a,4b können an ihren Enden halbkugelförmig, zylindrisch, kegelförmig oder ähnlich ausgebildet sein. Die Vorsprünge können auch pyramidenförmig ausgebildet sein. Hierdurch wird es ermöglicht, die Klemme unter verschiedenen Winkeln zum Anlegeinstrument zu halten, wodurch auf die sonst übliche Modellvielfalt verzichtet werden kann.

Die Schraubenfeder 5 ist so vorgespannt, daß die Bedienungsbranchen 2a,2b mit vorbestimmter Kraft zusammengezogen werden und damit auch die Maulteile 1a,1b mit vorbestimmter Kraft gegeneinander gedrängt werden.

Zum Anlegen der Klemme wird ein übliches Anlegeinstrument zwischen die Bedienungsbranchen 2a,2b eingeführt. Das Anlegeinstrument weist den Vorsprüngen entsprechende Vertiefungen auf, in die die Vorsprünge eingreifen können.

Da das Anlegeinstrument die Bedienungsbranchen 2a,2b nicht von außen umgreifen muß, sondern mit seinen Schenkein zwischen die Bedienungsbranchen eingeführt wird, werden keine Teile der Klemme verdeckt, und es bewirkt das Anlegeinstrument keine Sichtbehinderung bei seiner Anwendung.

Fig.3 zeigt eine abgewandelte Ausführung, bei der die Enden 6a,6b der Schraubenfeder 6 sich zwischen den Bedienungsbranchen 2a,2b überkreuzen. Hierdurch kann auf die Bedienungsbranchen 2a,2b eine größere Schließkraft aufgebracht werden.

## Patentansprüche

1. Klemme zum Abklemmen von Blutgefäßen oder Aneurismen, bestehend aus zwei ein Maulteil (1a,1b) und eine Bedienungsbranche (2a,2b) aufweisenden Armen, welche mit einer die Maulteile (1a,1b) gegeneinander drängenden, an den Bedienungsbranchen (2a,2b) befestigten Schließfeder (6) verbunden sind, wobei die Arme einander schwenklagerfrei überkreuzen und die Schließfeder eine Schraubenfeder (6) mit wenigstens einer Windung ist, deren verlängerte Federenden (6a,6b) je mit einer der Bedienungsbranchen (2a,2b) fest verbunden sind,
**dadurch gekennzeichnet, daß**
die Bedienungsbranchen (2a,2b) mit Rastelementen (4a,4b) in Form von Vorsprüngen oder Vertiefungen für ein Anlegeinstrument an den einander zugewandten Seiten der Bedienungsbranchen (2a,2b) versehen sind und daß die Schließfeder (6) zwischen den Bedienungsbranchen (2a,2b) liegt und mit ihren verlängerten Federenden (6a,6b) je mit einer der beiden Innenflächen der Bedienungsbranchen (2a,2b) verbunden sind.

2. Klemme nach Anspruch 1, **dadurch gekennzeichnet, daß**
sich die Enden (6a,6b) der Schließfeder (6) im Anschluß an die Federwindung (6) und vor der Befestigung an den Bedienungsbranchen (2a,2b) überkreuzen.

## Claims

1. A clip for clamping of blood vessels or aneurysms, consisting of two arms each having a bit portion (1a, 1b) and a handling segment (2a, 2b) and being connected to a closing spring (6) attached to the handling segments (2a, 2b) and urging the bit portions (1a, 1b) towards one another, the arms crossing one another without a swivel bearing and the closing spring being a coil spring (8) having at least one winding whose extended spring ends (6a, 6b) are rigidly connected to either one of the handling segments (2a, 2b), characterized in that on the sides of the handling segments (2a, 2b) facing one another the handling segments (2a, 2b) are provided with engaging elements (4a, 4b) in the form of protrusions or recesses for a clip-on instrument and that the closing spring (6) is located between the handling segments (2a, 2b) and connected through its extended spring ends (6a, 6b) to either one of the two inner faces of the handling segments (2a, 2b).

2. A clip as claimed in claim 1, characterized in that following the spring winding (6) and before being attached to the handling segments (2a, 2b) the ends (6a, 6b) of the closing spring (6) cross one another.

## Revendications

1. Pince pour vaisseaux sanguins et anévrismes comportant deux bras présentant une bouche (1a. 1b) et une branche pour le maniement (2a, 2b), ces bras étant reliés à un ressort de fermeture (8) fixé sur les branches de maniement (2a, 2b) et pressant l'une vers l'autre les bouches (1a, 1b), les bras se croisant sans élément de pivotement et le ressort de fermeture étant un ressort à boudin (6) présentant au moins une spire et dont chacune des deux extrémités prolongées (6a, 6b) est reliée fixement à une des branches de maniement (2a, 2b), caractérisée en ce que les branches de maniement (2a, 2b) sont pourvues de moyens d'enclenchement (4a, 4b), pour la pose d'un instrument, sous forme d'avancements ou de creux sur les côtés se faisant face des branches de maniement (2a, 2b) et en ce que le ressort de fermeture (6) est placé entre les branches de maniement (2a, 2b) et est relié par ses extrémités prolongées (6a, 6b) avec une des deux surfaces intérieures des branches de maniement (2a, 2b).

2. Pince selon la revendication 1, caractérisée en ce que les extrémités (6a, 6b) du ressort de fermeture (6) se croisent immédiatement après la spire (6) et avant la fixation sur les branches de maniement (2a, 2b).
